(19) Europäisches Patentamt
European Patent Office
Office européen des brevets

(11) Veröffentlichungsnummer: **0 344 114 B1**

(12) **EUROPÄISCHE PATENTSCHRIFT**

(45) Veröffentlichungstag der Patentschrift: **06.04.94**

(51) Int. Cl.$^5$: **C07D 211/46**, C08K 5/34

(21) Anmeldenummer: **89810369.2**

(22) Anmeldetag: **18.05.89**

Die Akte enthält technische Angaben, die nach dem Eingang der Anmeldung eingereicht wurden und die nicht in dieser Patentschrift enthalten sind.

(54) **Ungesättigte Derivate von 2,2,6,6-Tetramethylpiperidin.**

(30) Priorität: **27.05.88 CH 2007/88**

(43) Veröffentlichungstag der Anmeldung:
**29.11.89 Patentblatt 89/48**

(45) Bekanntmachung des Hinweises auf die Patenterteilung:
**06.04.94 Patentblatt 94/14**

(84) Benannte Vertragsstaaten:
**DE FR GB IT**

(56) Entgegenhaltungen:
**DE-A- 2 040 983**
**DE-A- 2 258 752**

(73) Patentinhaber: **CIBA-GEIGY AG**
**Klybeckstrasse 141**
**CH-4002 Basel(CH)**

(72) Erfinder: **Rytz, Gerhard, Dr.**
**Am Bach 2**
**CH-3150 Schwarzenburg(CH)**
Erfinder: **Slongo, Mario, Dr.**
**Sägetrainweg 553**
**CH-1712 Tafers(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft neue Verbindungen, welche ungesättigte Derivate von 2,2,6,6-Tetramethylpiperidin sind, sowie deren Verwendung als Lichtschutzmittel und Vernetzer.

Es handelt sich dabei um die Verbindungen der Formel I

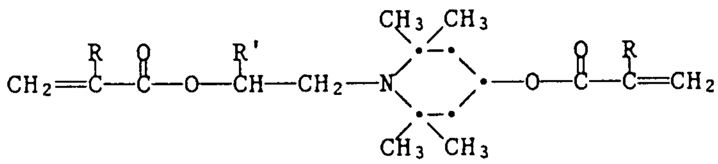

I

worin R Wasserstoff, Methyl oder Cyan ist und R' Wasserstoff, $C_1$-$C_8$-Alkyl oder Phenyl ist.

Es ist bekannt, dass Derivate des Tetramethylpiperidins als Lichtschutzmittel verwendet werden können, insbesondere in Polymeren. Tetramethylpiperidinderivate, die in 1- und 4-Stellung substituiert sind, sind in der DE-A-2 258 752 offenbart.

In der DE-A-2 040 983 sind auch bereits Acrylsäurederivate des 2,2,6,6-Tetramethyl-4-piperidinols beschrieben worden, die sich durch Copolymerisation in Polymere einbauen lassen. In der EP-A-101 411 sind s-Triazinderivate beschrieben, die neben Tetramethylpiperidingruppen mehrere ethylenisch ungesättigte Gruppen besitzen und die sich zum Vernetzen von Polyolefinen eignen.

Die Verbindungen der Formel I eignen sich zur Herstellung vernetzter Polymere durch Copolymerisation mit ethylenisch ungesättigten Monomeren.

Bevorzugt sind Verbindungen der Formel I, worin R und R' unabhängig voneinander H oder Methyl sind.

Beispiele für Verbindungen der Formel I sind:
1-(2-Acryloyloxyethyl)-4-acryloyloxy-2,2,6,6-tetramethylpiperidin
1-(2-Methacryloyloxyethyl)-4-methacyrloyloxy-2,2,6,6-tetramethylpiperidin
1-[2-(α-Cyanacryloyloxy)ethyl]-4-(α-cyanacryloyloxy)-2,2,6,6-tetramethylpiperidin
1-(2-Acryloyloxypropyl)-4-acryloyloxy-2,2,6,6-tetramethylpiperidin
1-(2-Methacryloyloxybutyl)-4-methacryloyloxy-2,2,6,6-tetramethylpiperidin
1-(2-Acryloyloxy-2-phenylethyl)-4-acryloyloxy-2,2,6,6-tetramethylpiperidin
Die Verbindungen der Formel I können aus den Diolen der Formel II

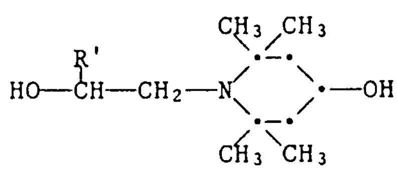

II

durch Acylierung beider OH-Gruppen mit den Säuren $CH_2 = C(R)$-COOH oder deren Estern oder Säurechloriden nach an sich bekannten Methoden hergestellt werden. Bevorzugt verwendet man die Ester niedriger Alkohole $CH_2 = C(R)$-COO($C_1$-$C_4$-Alkyl) unter Mitverwendung eines Umesterungskatalysators. Als Umesterungskatalysator eignen sich z.B. Tetraalkyltitanate, Alkalimetallalkoxide oder Alkalimetallamide. Zur Verhinderung einer Polymerisation während der Herstellung setzt man vorzugsweise kleinere Mengen eines Polymerisationsinhibitors zu. Die Isolierung bzw. Reinigung der Produkte kann durch die üblichen Verfahren geschehen, wie z.B. durch Kristallisation, Hochvakuumdestillation oder Chromatographie. Die Verbindungen der Formel II sind bekannte Verbindungen und z.B. in der DE-A-2 352 658 beschrieben.

Eine alternative Herstellungsmethode besteht darin, dass man ein Diol der Formel II mit einer $\beta$-Halogencarbonsäure verestert und den Ester anschliessend durch Umsetzung mit einer Base dehydrohalogeniert.

Die Verbindungen der Formel I können als Lichtschutzmittel für organische Polymere verwendet werden. Beispiele hierfür sind die folgenden Polymeren.

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte

(LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-($\alpha$-methylstyrol).

5. Copolymere von Styrol oder $\alpha$-Methylstyrol mit Dienen oder Acryl-derivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder $\alpha$-Methylstyrol, wie z.B. Styrol auf Polybutadien; Styrol auf Polybutadien-Styrol- oder Polybutadien-Acrylnitril-Copolymere; Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien; Styrol und Alkylacrylate bzw. Alkylmethacrylate auf Polybutadien; Styrol und Acrylnitril auf Ethylen-Propylen-Dien-Terpolymeren; Styrol und Acrylnitril auf Polyalkylacrylaten oder Polyalkylmethacrylaten; Styrol und Acrylnitril auf Acrylat-Butadien-Copolymeren; sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.b. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10,

6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Die Verbindungen der Formel I werden dabei vorzugsweise in einer Menge von 0,01 bis 5 Gew.-%, insbesondere 0,05 bis 2 Gew.-%, bezogen auf das zu stabilisierende Polymer, zugesetzt. Der Zusatz kann durch Vermischen mit dem Polymeren in irgendeiner Phase der Verarbeitung erfolgen. Ausser der Verbindung der Formel I können dem Polymeren noch andere Stabilisatoren oder sonstige Additive zugesetzt werden wie z.B. die folgenden Additive:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-($\alpha$-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2′-Thio-bis-(6-tert.-butyl-4-methylphenol), 2,2′-Thio-bis-(4-octylphenol), 4,4′-Thio-bis-(6-tert.butyl-3-methylphenol), 4,4′-Thio-bis-(6-tert.butyl-2-methylphenol).

1.4. Alkyliden-Bisphenole, z.B. 2,2′-Methylen-bis-(6-tert.butyl-4-methylphenol), 2,2′-Methylen-bis-(6-tert.butyl-4-ethylphenol), 2,2′-Methylen-bis-[4-methyl-6-($\alpha$-methylcyclohexyl)-phenol], 2,2′-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-($\alpha$-methylbenzyl)-4-nonylphenol], 2,2'-Methylen-bis-[6-($\alpha$,$\alpha$-dimethylbenzyl)-4-nonylphenol], 4,4'-Methylen-bis-(2,6-di-tert.butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)-butyrat],Bis-(3-tert.butyl-4-hydroxy-5-methylphenyl)-dicyclopentadien, Bis-[2-(3'-tert.butyl-2'-hydroxy-5'-methyl-benzyl)-6-tert.butyl-4-methyl-phenyl]-terephthalat.

1.5 Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiolterephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxy-stearinsäureanilid, 2,4-Bis-(octylmercapto)-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.9. Ester der β-(3,5-Dicyclohexyl-4-hydroxyphenyl)-propionsäure mit ein- oder oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.10. Amide der β-(3,5-Di-tert.butyl-4-hydroxyphenyl)-propionsäure, wie z.B. N,N′-Bis-(3,5-di-tert.butyl-4-hydroxyphenylpropionyl)-hexamethylendiamin, N,N′-Bis-(3,5-di-tert.butyl-4-hydroxyphenylpropionyl)-trimethylendiamin, N,N′-Bis-(3,5-di-tert.butyl-4-hydroxyphenylpropionyl)-hydrazin.

2. UV-Absorber und Lichtschutzmittel

2.1. 2-(2′-Hydroxyphenyl)-benztriazole, wie z.B. das 5′-Methyl-, 3′,5′-Di-tert.butyl-, 5′-tert.Butyl-, 5′-(1,1,3,3-Tetramethylbutyl)-, 5-Chlor-3′,5′-di-tert.butyl-, 5-Chlor-3′-tert.butyl-5′-methyl-, 3′-sec.Butyl-5′-tert.butyl, 4′-Octoxy-, 3′,5′-Di-tert.amyl-, 3′,5′-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2′,4′-Trihydroxy-, 2′-Hydroxy-4,4′-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β,-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methylindolin.

2.5. Nickelverbindungen, wie z.B. Nickelkomplexe des 2,2′-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäuremonoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N′-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1′-(1,2-Ethandiyl)-bis-(3,3,5,5-tetramethyl-piperazinon).

2.7. Oxalsäurediamide, wie z.B. 4,4′-Di-octyloxy-oxanilid, 2,2′-Di-octyloxy-5,5′-di-tert.butyl-oxanilid, 2,2′-Di-dodecyloxy-5,5′-di-tert.butyl-oxanilid, 2-Ethoxy-2′-ethyl-oxanilid, N,N′-Bis-(3-dimethylaminopropyl)-oxalamid, 2-Ethoxy-5-tert.butyl-2′-ethyloxanilid und dessen Gemisch mit 2-Ethoxy-2′-ethyl-5,4′-di-tert.butyl-oxanilid, Gemische von o-und p-Methoxy- sowie von o- und p-Ethoxy-di-substituierten Oxaniliden.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrittetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Bevorzugt verwendet man die Verbindungen der Formel I zur Copolymerisation mit anderen ethylenisch ungesättigten Monomeren unter Bildung vernetzter Copolymerisate. Beispiele solcher copolymerisierbarer Monomere sind Derivate von Acryl-, Methacryl-, Croton- oder Maleinsäure (insbesondere deren Ester, Amide und Nitrile), Vinylester von aliphatischen Carbonsäuren, Vinylchlorid, Vinylidendichlorid, Styrol, α-Methylstyroloder N-Vinylpyrrolidon.

Die Copolymerisate können auch zwei oder mehrere solcher ungesättigter Monomere enthalten. Sie können auch Monomere mit zwei oder mehreren ethylenischen Doppelbindungen enthalten, die als Vernetzer fungieren. Beispiele solcher Vernetzer sind vor allem Polyol-poly(meth)acrylate wie z.B. Ethylenglykol-diacrylat, Butandiol-dimethacrylat oder Pentaerythrittetraacrylat.

Gegenstand der Erfindung sind daher auch vernetzte Copolymerisate, erhältlich aus (a) mindestens einem ethylenisch ungesättigten Monomer und (b) einer Verbindung der Formel I. Die Verbindung der Formel I ist darin vorzugsweise in einer Menge von 0,5 bis 10 Gew.-% enthalten.

Geschieht diese Copolymerisation unter dispergierenden Bedingungen in einem inerten Lösungsmittel, in dem das Copolymer unlöslich ist, so erhält man eine Polymerdispersion, die aus vernetzten Mikropartikeln besteht. Diese Mikropartikeln können zur Herstellung von Anstrichstoffen mit hohem Festkörperanteil verwendet werden, wie dies beispielsweise in der EP-A-003 166 beschrieben ist.

Die Mitverwendung von Verbindungen der Formel I als Comonomere bei der Herstellung solcher Polymer-Mikropartikel hat zwei Vorteile. Erstens dient die difunktionelle Piperidinverbindung als Vernetzer. Zweitens wird dadurch eine Stabilisierung der Mikropartikel gegen Licht, insbesondere gegen UV-Licht bewirkt. Damit wird auch eine Stabilisierung von Anstrichstoffen bewirkt, in denen solche Mikropartikeln enthalten sind.

Die Anstrichstoffe enthalten die Mikropartikel als disperse Phase in einer flüssigen Phase von Bindemittel und Lösungsmittel dispergiert. Bei der thermischen Härtung solcher Anstrichstoffe kann das Lichtschutzmittel nicht verdampfen, da es in den Mikropartikeln chemisch gebunden ist. Auch im Gebrauch solcher Anstriche kann das Lichtschutzmittel nicht durch Elution oder Migration verlorengehen.

Die Copolymerisate der Verbindungen der Formel I können aber nicht nur als Mikropartikel für festkörperreiche Anstrichstoffe verwendet werden sondern auch zu sonstigen Verwendungszwecken, bei denen ein vernetztes Polymer benötigt wird.

Die folgenden Beispiele illustrieren die Herstellung der erfindungsgemässen Verbindungen und ihre Verwendung. Darin bedeuten Teile Gewichtsteile und Prozente Gewichtsprozente, soweit nicht anders angegeben.

Beispiel 1: 1-(2-Methacryloyloxyethyl)-4-methacryloyloxy-2,2,6,6-tetramethylpiperidin

201,3 g 1-(2-Hydroxyethyl)-4-hydroxy-2,2,6,6-tetramethylpiperidin, 360,4 g Methylmethacrylat, 1,5 g 2,6-Di-tert-butyl-p-kresol und 0,4 g N,N'-Bis[β-(3,5-di-tert.butyl-4-butyl-4-hydroxyphenyl)-propionyl]-hexamethylendiamin werden unter Stickstoff bei 110 °C solange erhitzt, bis durch eine Vigreux-Kolonne (40 cm wirksame Länge, mit Doppelmantel, verspiegelt und evakuiert) 20 ml Methylmethacrylat abdestilliert sind. Man lässt auf 85 °C abkühlen, gibt 2,4 ml Tetrabutyltitanat zu und erhitzt so, dass ganz langsam Methanol abdestilliert (Badtemperatur 110 °C, Kopftemperatur 60 °C). Nach 2 Stunden gibt man 80,1 g Methylmethacrylat zu, lässt 14 Stunden weiterreagieren (Badtemperatur 90 °C), gibt 1,2 ml Tetrabutyltitanat zu und lässt unter denselben Bedingungen weitere 24 Stunden reagieren. Ueberschüssiges Methylmethacrylat wird abdestilliert. Das verbleibende Rohprodukt wird an einer $SiO_2$-Säule chromatographisch gereinigt (Laufmittel: Ether: Methanol (mit $NH_3$ gesättigt) = 9:1). Man erhält reines 1-(2-Methacryloyloxyethyl)-4-methacryloyloxy-2,2,6,6-tetramethylpiperidin als farbloses Oel. $n_D^{20}$ = 1,485.

| | | | | |
|---|---|---|---|---|
| Analyse: | Ber.<br>Gef. | C = 67,63 %<br>C = 67,61 % | H = 9,26 %<br>H = 9,22 % | N = 4,15 %<br>N = 4,04 % |

Beispiel 2: 1-(2-Acryloyloxyethyl)-4-acryloyloxy-2,2,6,6-tetramethylpiperidin

Verwendet man anstelle von Methylmethacrylat Butylacrylat und verfährt im übrigen wie in Beispiel 1, so erhält man 1-(2-Acryloyloxyethyl)-4-acryloyloxy-2,2,6,6-tetramethylpiperidin als farbloses Oel.

| | | | | |
|---|---|---|---|---|
| Analyse: | Ber.<br>Gef. | C = 66,0 %<br>C = 67,7 % | H = 8,8 %<br>H = 9,0 % | N = 4,5 %<br>N = 4,6 % |

Beispiel 3: 1-(2-Acryloyloxypropyl)-4-acryloyloxy-2,2,6,6-tetramethylpiperidin

Zu 64,6 g 1-(2-Hydroxypropyl)-4-hydroxy-2,2,6,6-tetramethylpiperidin, 181,8 g Triethylamin und 300 ml Toluol werden unter Rühren und Kühlen mit einem Eisbad während 1 Stunde 91,4 g 3-Chlorpropionsäurechlorid, vermischt mit 60 ml Toluol, so zugetropft, dass die Temperatur nicht über 60 °C steigt. Dann wird während 3,5 Stunden auf 60 °C erhitzt. Nach Zugabe von 9,1 g 3-Chlorpropionsäurechlorid wird eine weitere Stunde bei 60 °C gerührt, dann lässt man abkühlen, filtriert und wäscht die flüssige Phase viermal mit 150 ml Wasser. Nach Trocknen und Eindampfen wird der Rückstand in Hexan/Aceton gelöst und an einer $SiO_2$-Säule chromatographisch gereinigt. Dabei erhält man reines 1-(2-Acryloyloxypropyl)-4-acryloyloxy-2,2,6,6-tetramethylpiperidin als leicht gelbliches Oel mit $n_D^{20}$ = 1,486 (Stickstoffgehalt berechnet: 4,33 %; gefunden 4,17 %).

Beispiel 4: 1-(2-Methacryloyloxypropyl)-4-methacryloyloxy-2,2,6,6-tetramethylpiperidin

Verwendet man anstelle von 1-(2-Hydroxyethyl)-4-hydroxy-2,2,6,6-tetramethylpiperidin, 1-(2-Hydroxypropyl)-4-hydroxy-2,2,6,6-tetramethylpiperidin und verfährt im übrigen wie in Beispiel 1, wobei am Schluss in Toluol gelöst und an einer $SiO_2$-Säule chromatographisch gereinigt wird, so erhält man reines 1-(2-Methacryloyloxypropyl)-4-methacryloyloxy-2,2,6,6-tetramethylpiperidin als leicht gelbliches Oel mit $n_D^{20}$ = 1,482 (Stickstoffgehalt berechnet: 3,99 %; gefunden 3,88 %).

## Patentansprüche

1. Eine Verbindung der Formel I,

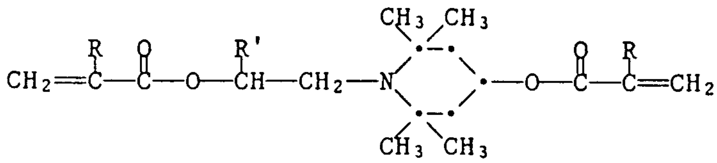

I

worin R Wasserstoff, Methyl oder Cyan ist und R' Wasserstoff, $C_1$-$C_8$-Alkyl oder Phenyl ist.

2. Eine Verbindung gemäss Anspruch 1 der Formel I, worin R und R' unabhänging voneinander H oder Methyl sind.

3. 1-(2-Acryloyloxyethyl)-4-acryloyloxy-2,2,6,6-tetramethylpiperidin als Verbindung gemäss Anspruch 1.

4. 1-(2-Methacryloxyethyl)-4-methacryloyloxy-2,2,6,6-tetramethylpiperidin als Verbindung gemäss Anspruch 1.

5. Organisches Polymer, enthaltend als Stabilisator eine Verbindung der Formel I gemäss Anspruch 1.

6. Organisches Polymer gemäss Anspruch 6, enthaltend 0,01 bis 5 Gew.-%, bezogen auf das Polymer, einer Verbindung der Formel I.

7. Vernetztes Copolymerisat, erhältlich aus (a) mindestens einem ethylenisch ungesättigten Monomer und (b) einer Verbindung der Formel I des Anspruches 1.

8. Vernetztes Copolymerisat gemäss Anspruch 7, enthaltend 0,5 - 10 Gew.-% der Komponente (b).

## Claims

1. A compound of the formula I

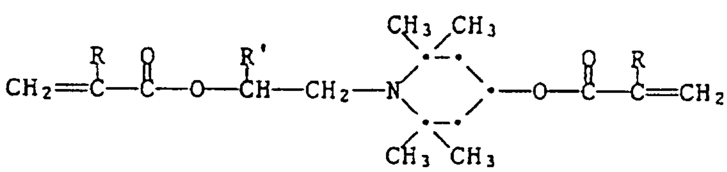

I

wherein R is hydrogen, methyl or cyano, and R' is hydrogen, $C_1$-$C_8$alkyl or phenyl.

2. A compound according to claim 1 of the formula I, wherein R and R' are each independently of the other hydrogen or methyl.

3. 1-(-2-Acryloyloxyethyl)-4-acryloyloxy-2,2,6,6-tetramethylpiperidine as a compound according to claim 1.

4. 1-(2-Methacryloyloxyethyl)-4-methacryloyloxy-2,2,6,6-tetramethylpiperidine as a compound according to claim 1.

5. An organic polymer comprising as stabilizer a compound of the formula I according to claim 1.

6. An organic polymer according to claim 5, containing 0.01 to 5 % by weight, based on the polymer, of a compound of the formula I.

7. A crosslinked copolymer obtainable from (a) at least one ethylenically unsaturated monomer and (b) a compound of the formula I according to claim 1.

8. A crosslinked copolymer according to claim 7, containing 0.5 to 10 % by weight of component (b).

**Revendications**

1. Composé de formule I

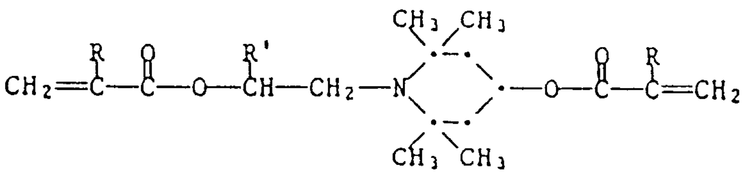

I

dans laquelle R représente un atome d'hydrogène, un groupe méthyle ou un groupe cyano, et R' représente un atome d'hydrogène, un groupe alkyle en $C_1$-$C_8$ ou un groupe phényle.

2. Composé selon la revendication 1 de formule I, dans laquelle R et R' représentent, indépendamment l'un de l'autre, un atome d'hydrogène ou un groupe méthyle.

3. Composé selon la revendication 1 consistant en la 1-(2-acryloyloxyéthyl)-4-acryloyloxy-2,2,6,6-tétraméthylpipéridine.

4. Composé selon la revendication 1, caractérisé en ce qu'il consiste en la 1-(2-méthacryloyloxyéthyl)-4-méthacryloyloxy-2,2,6,6-tétraméthylpipéridine.

5. Polymère organique, contenant comme stabilisant un composé de formule I selon la revendication 1.

6. Polymère organique selon la revendication 6, contenant de 0,01 à 5 % en poids par rapport au polymère d'un composé de formule I.

7. Copolymérisat réticulé, obtenu à partir de (a) au moins un monomère à insaturation éthylénique et (b) un composé de formule I selon la revendication 1.

8. Copolymérisat réticulé, selon la revendication 7, contenant de 0,5 à 10 % en poids du composant (b).